(19)

Europäisches
Patentamt
European
Patent Office
Office européen
des brevets

(11)     **EP 3 620 775 B1**

(12)                    **EUROPEAN PATENT SPECIFICATION**

(45) Date of publication and mention
of the grant of the patent:
**16.06.2021  Bulletin 2021/24**

(51) Int Cl.:
***G01N 21/33*** *(2006.01)*        ***G01N 21/61*** *(2006.01)*
***G01N 21/85*** *(2006.01)*        *G01N 21/17* *(2006.01)*

(21) Application number: **19195698.6**

(22) Date of filing: **05.09.2019**

(54) **DEVICE FOR TOMOGRAPHIC MEASUREMENT OF GAS DISTRIBUTION ON A TEST BENCH**

VORRICHTUNG ZUR TOMOGRAFISCHEN MESSUNG DER GASVERTEILUNG AUF EINEM
PRÜFSTAND

DISPOSITIF DE MESURE TOMOGRAPHIQUE DE DISTRIBUTION DE GAZ SUR UN BANC D'ESSAI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **07.09.2018  GB 201814591**

(43) Date of publication of application:
**11.03.2020  Bulletin 2020/11**

(73) Proprietor: **Virtual Vehicle Research GmbH
8010 Graz (AT)**

(72) Inventors:
  • **Fischbacher, Bernhard
8010 Graz (AT)**
  • **Lechner, Bernhard
8010 Graz (AT)**
  • **Brandstätter, Bernhard
8010 Graz (AT)**

(74) Representative: **Dilg, Haeusler, Schindelmann
Patentanwaltsgesellschaft mbH
Leonrodstraße 58
80636 München (DE)**

(56) References cited:
**WO-A1-91/18280          CN-A- 106 290 244
DE-A1-102012 200 107**

  • **FELIX STRITZKE ET AL: "Ammonia
concentration distribution measurements in the
exhaust of a heavy duty diesel engine based on
limited data absorption tomography", OPTICS
EXPRESS, vol. 25, no. 7, 30 March 2017
(2017-03-30) , pages 8180-8191, XP055662529,
DOI: 10.1364/OE.25.008180**
  • **SONG JUNLING ET AL: "Tomography system for
measurement of gas properties in combustion
flow field", CHINESE JOURNAL OF
AERONAUTICS, ELSEVIER, AMSTERDAM, NL,
vol. 30, no. 5, 22 August 2017 (2017-08-22), pages
1697-1707, XP085213859, ISSN: 1000-9361, DOI:
10.1016/J.CJA.2017.08.004**

## Description

Background of the invention

The NOX emission reduction of diesel engines is still an important issue in engine development. The introduction of the current EURO 6c or 6d temp and upcoming emission legislations including the new real driving emission (RDE) cycle forces manufacturers to radically reduce NOX emission. The most promising technology for exhaust aftertreatment is the selective catalytic reduction (SCR). SCR technology uses a urea-water solution (UWS) being injected into the exhaust gas. The UWS is converted to ammonia through thermolysis and hydrolysis and acts as reduction agent for NOX in the SCR reaction. The ammonia concentration distribution entering the catalyst converter is a key parameter for best conversion performance. Commonly used methods extract gas from various discrete positions in the cross section which leads to poor temporal resolution and measurement uncertainties due to depositions and chemical reactions along the analysis path. This new approach enables the investigation of dynamic processes in the exhaust aftertreatment system to increase the system performance.

Art Background

In-situ optical measurement techniques are not intrusive and therefore offer the great advantage of not manipulating the original experiment. In addition, the measurement quantity is measured directly at the point of interest. For ammonia detection various measurement techniques are available (e.g. Hole, O.M.; Measuring ammonia - Development and application of measurement techniques for the detection of ammonia, Master thesis, University of Lund, 2012). Within absorption spectroscopy the light absorption by the gas of intertest is wavelength specific. By detecting the light absorption at the sensitive wavelengths with possibly no other gas species absorbing in this spectral range the line concentration can be determined. The concentration estimation follows the Beer-Lambert absorption law (Eq. 1). The absorbance A is defined by the ratio of the incident light and the detected light and is used to calculate the line concentration c using the calibrated absorption coefficient $\alpha$ and the path length I (Eq. 1). Since the sampling rate of spectrometers is limited by the integration time of the detector and hence the intensity of the light source, the developed measurement system applies non-dispersive techniques using an optical bandpass filter in the desired spectral range in combination with a photodiode.

$$A = \ln\left(\frac{I_0}{I}\right) = \alpha \cdot c \cdot l$$

The line concentration per optical path represents the integral of gas concentration along the path. By applying as many optical paths as possible in different positions and angles through the cross section the information gain can be increased only limited by the optical access to the cross section. In this application 20 light paths are instrumented through the cross section. The resulting inverse problem is reconstructed by appliance of Least Squares regression and Tikhonov regularization (e.g. Daun, K. J.; Infrared species limited data tomography through Tikhonov reconstruction, Journal of Quantitative Spectroscopy and Radiative Transfer, 2010, Vol. 111, 105 - 115, DOI: 10.1016/j.jqsrt.2009.08.003) leading to 2D images of the ammonia concentration distribution in the measurement cross section.

Several inventions and approaches to measure the gas distribution in a gas flow are presented:

US 6,593,573 B1 describes an apparatus for monitoring the distribution of a chemical species with a plurality of radiation sources and radiation detectors around the perimeter of a vessel.

US 2010/0241361 A1 presents a method of absorption spectroscopy with spatial resolution based on data at multiple wavelengths along multiple line of sight paths through a media.

US 2015/0355080 A1 describes a system for species concentration reconstruction for an exhaust system including emitter, detector and controller.

CN 104280355 presents a detecting device and method for the concentration measurement of ammonia gas and sulfur dioxide gas, which consists of an ultraviolet light source, a collimator lens, a runner, a gas absorption cell, a condensing lens, an ultraviolet fibre, a spectrometer and a computer.

CN 106442403 presents an ammonia pollution detection system, which comprises a photoelectric device unit and a gas path unit and is particularly suited to determine the gas concentration in SCR systems.

KR 101839704 B1 presents a method to use multiple beams through a measurement layer using TDLAS in the NIR to simultaneously measure temperature and gas concentration distribution in a pipe. The method is based on MART technology, N-point algorithm and Least Squares.

WO 2009/052157 A1 presents an apparatus and methods for species concentration within a jet engine plume. The apparatus uses TDLAS with one or multiple beams in the NIR being traversed or rotated across a measurement section of interest. The methods comprise algorithms to obtain the concentration along the beams and how to move the emitter/detector array across the section of interest.

US 5,798,840 describes a fast absorption optical tomography instrument capable of generating 100 projections of 100 elements each in less than 200 ns.

J. Song et al., "Tomography system for measurement of gas properties in combustion flow field", Chinese J. Aeronaut., 30(5), 1697-1707, 2017, discloses a system for reconstruction of temperature and gas concentration with both spatial and temporal resolutions. Optical paths are provided by tomography frames mounted on linear stages and a rotary stage allowing to rotate the optical measurement paths incrementally around the area of interest.

Summary of the Invention

The present invention provides a device for gas concentration measurement based on tomographic principles with a novel feature of providing movable measurement layers, as defined in claim 1.

The invention with the rotatable layers is used for optimized projections in order to improve tomographic reconstruction. According to the expected gas distribution, the most suitable angle between the two layers can be chosen.

The present innovation provides a device to determine the concentration in a gas. One specific aim of the invention is to allow for ammonia concentration measurement in SCR applications.

Ammonia absorbs light mainly in three spectral ranges: in the DUV, near infrared and mid infrared region. The measurement specification identified the deep ultraviolet (DUV) spectral region to be the best choice. The developed measurement system must be capable of detecting the ammonia concentration before as well as after the SCR catalyst converter. The absorption coefficient defining the system sensitivity is highest in the DUV region and 10 times higher than in the MIR and 10000 time higher than in the NIR region. The cross sensitivity to nitric oxides and aromatic compounds is no issue for measurements on the hot gas test bench but must be considered for migration of the system to the engine test bench.

The properties of the three most sensitive ammonia absorption spectral ranges are listed in the table below:

| | **near infrared (NIR)** | **mid infrared (MIR)** | **deep ultraviolet (DUV)** |
|---|---|---|---|
| **spectral region** | $1.6\,\mu m$ | $10\,\mu m$ | $210$ nm |
| **absorption coefficient** | $10^{-21}$ cm$^2$ -- | $10^{-18}$ cm$^2$ o | $2*10^{-17}$ cm$^2$ +++ |
| **cost** | ++ | -- | + |
| **availability** | wide variety of materials at low price ++ | hardly any temperature resistant materials - | moderate price, spectral limit for fibers and filters o |
| **cross sensitivity** | $H_2O$ - | hot $CO_2$ + | NO, NO$_2$, -- |

To enable geometric localization of the gas concentration tomographic methods are applied. Reconstruction quality is strongly dependent on the number of projections and their intelligent arrangement. To choose the best optical beam arrangement, several scenarios were investigated and tested for their information content using randomized test data.

The invention comprises:

- Two sections of an inner pipe 1a and 1b which have the same dimensions and the same axis and can be rotated relative to each other
- Several holes aligned in two lines 2a and 2b in each inner pipe 1a and 1b
- Optical lenses that allow light to pass through the lines of holes 2a and 2b from outside the pipe, pass the cross section of the pipe and pass the opposite holes

- Two outer rings 3a and 3b with light sources (typically optical fibres) and sensors (typically photodiodes with filters)
- Two mounting rings 4a and 4b that connect the two halves of the inner pipe 1a and 1b and allow to rotate one or both inner pipes 1a and 1b with discrete angles relative to each other according to the discrete positions of the holes in the line of holes 2a and 2b

In one specific implementation of the invention, the measurement path of the optical beams consists of a deuterium gas discharge lamp as light source for seven optical beams connected via a multifurcated solarization resistant UV fiber-optic cable. The optical access to the measurement cross section is realized via fused silica lenses acting as collimation lens for the fiber. On the detector side of the beam a fused silica lens is used as converging lens focusing the light on the detector. The detector consists of an optical bandpass filter and a photodiode directly connected to an optimized transimpedance amplifier (TIV). The bandpass filter has a central wavelength of 205 nm with a FWHM of 10 nm. Ammonia absorbs in the region of 200 nm to 225 nm meaning that the filter forms an integral over the spectral range of interest and hence realizes the non-dispersive measurement principle. The properties of the UV enhanced photodiode were considered designing the TIV with respect to amplification, speed and stability. The cross section is scanned with a regular beam arrangement leading to 20 line concentration time signals. After applying reconstruction algorithms on the time signals, a 2D image for each sample in the time domain is obtained.

The inverse problem of limited data hard field tomography can be solved by appliance of Least Squares regression and Tikhonov regularization (Eq. 2). The formulation consists of the minimization of a measurement term and a regularization term. The regularization term consists of a weighting factor $\lambda$ and a regularization matrix L, here a discrete Laplacian (Gradient) operator to introduce smoothness as a prior information. The introduction and correct weighting of prior information are key factors for the reconstruction quality of the results. The best tradeoff between extinction of artefacts and over-smoothing has to be found to minimize reconstruction error.

$$x_{reconst} = \mathrm{argmin}_{x>0}\{\|A \cdot x - b\|^2 + \|\lambda \cdot L \cdot x\|^2\} \qquad (2)$$

Summarizing, the invention comprises:

- Two sections of a pipe (one section forming a first inner pipe and a further section forming a second inner pipe), wherein the inner pipes have the same dimensions and the same common axis and can be rotated (along its circumferential direction) relative to each other
- Several holes in the respective inner pipes to form paths for multiple light beams from light sources through the inner pipes to the detectors
- Optical lenses that allow light to pass through the (lines of) holes from outside the respective inner pipe, pass the cross section of the respective inner pipe and pass the opposite holes of the respective inner pipe
- Two outer rings with light sources (typically optical fibres) and sensors (typically photodiodes with filters)

Two mounting rings 4a and 4b that connect the inner pipes 1a and 1b and allow to rotate one or both inner pipes 1a and 1b together with the respective outer rings 3a and 3b with discrete angles relative to each other according to the discrete positions of the mounting holes in the mounting rings 4a and 4b in order to optimize projections and improve tomographic reconstruction. Accordingly, according to an aspect of the present invention, a device to determine the gas concentration distribution in a flow based on non-dispersive absorption spectroscopy is provided, as defined in claim 1. The device comprises two optical measurement layers providing different positions for optical paths that can be rotated relative to each other with discrete angles between the two layers, each measurement layer consisting of one inner pipe 1a and 1b with optical access via several holes 2a and 2b and lenses to form multiple light beams from light sources through the inner pipe to the detectors one outer ring 3a and 3b with light sources and sensors arranged around the inner pipe 1a and 1b allowing light to pass the cross section of the inner pipe 1a and 1b.

Furthermore, the device comprises two mounting rings 4a and 4b that connect the two inner pipes 1a and 1b and fallow to rotate one or both inner pipes 1a and 1b together with the outer rings 3a and 3b with discrete angles relative to each other according to the discrete positions of the mounting holes 5a and 5b in the mounting rings 4a and 4b in order to optimize projections and improve tomographic reconstruction.

In other words, the device comprises two optical measurement layers providing different paths for optical light beams, wherein the layers (i.e. the first and second inner pipe including one of the respective layers) can be rotated relative to each other with discrete angles between respective light beams within the respective layers.

An optical measurement layer is defined by a two-dimensional plane in which respective light beams extend.

And optical measurement layer may be defined as a cross-sectional plane of a respective inner pipe, wherein the cross-sectional plane comprises a normal being perpendicular to the radial direction and the circumferential direction and being parallel to the axial direction of the respective inner pipe.

Each measurement layer consists of and can be provided by a respective pipe. A first inner pipe forms a first optical measurement layer and a second inner pipe forms a second optical measurement layer. The first optical measurement layer is parallel but spaced apart with respect to the second optical measurement layer.

For forming the respective optical measurement layers, each of the respective first inner pipe and second inner pipe comprises respective holes for providing an optical access through the respective first and second inner pipes and the respective layers. Furthermore, respective lenses are provided in the path of the light beams. Hence, through the holes, multiple light beams are guided through the respective first and second inner pipes within the respective optical measurement layers from light sources through the respective first and second inner pipe to respective detectors.

For example, the first inner pipe comprises a line of holes, such that the holes are formed spaced apart along a circumferential direction of the first inner pipe. The circumferential direction is perpendicular to an axial direction of the respective pipe and to a radial direction of the respective pipe. Specifically, in the first inner pipe a first hole forms an inlet hole through which a respective light beam may enter the first inner pipe and a second hole, which is formed in the first inner pipe opposed to the first hole, forms an outlet hole through which the respective light beam, which entered the first inlet hole, exits the first inner pipe. Hence, a respective light source is arranged at or close to the first inlet hole, wherein a respective light sensor is arranged at or close to the first outlet hole for receiving the light beam which has been emitted by the light source through the first inlet hole.

Furthermore, a first outer ring and a second outer ring is provided. The first outer ring is mounted to the first inner pipe and the second outer ring is mounted to the second inner pipe. The respective outer rings are equipped with light sources and sensors arranged around (i.e. along the circumferential direction) the first and second inner pipe allowing light to pass the cross section of the inner pipe, in particular between an inlet hole and an opposing outlet hole.

Furthermore, the first mounting ring and the second mounting ring is provided. The two mounting rings connect and fix the two inner pipes and allows to rotate one or both inner pipes together with the outer rings with discrete angles relative to each other according to the discrete positions of respective mounting holes in the mounting rings in order to optimize projections and improve tomographic reconstruction.

In other words, the first outer ring to which the first inner pipe is mounted, can be fixed to the first mounting ring. The second outer ring, to which the second inner pipe is mounted, can be fixed to the second mounting ring. The first outer ring can be fixed to the first mounting ring with different predefined circumferential orientation. In other words, the first outer ring can be rotated in a predefined rotational orientation, the first outer ring together with the first inner pipe can be fixed to the first mounting ring.

Additionally or alternatively, the second outer ring can be fixed to the second mounting ring with different predefined circumferential orientation. Hence, the first outer ring and the second outer ring can be adjusted and orientated with respect to each other along its circumferential direction such that also both inner pipes may be rotated with respect to each other until a desired circumferential orientation of the respective pipes is fulfilled. Hence, the angles between a first light beam running through the first optical measurement layer and hence through a first pair of holes in the first inner pipe and a second light beam running through the second optical measurement layer and hence through a second pair of holes in the second inner pipe can be adjusted in order to optimize projections and improve tomographic reconstruction.

According to the present invention a device is provided to determine the gas concentration distribution in a flow based on non-dispersive absorption spectroscopy as defined in claim 1.

It has to be noted that embodiments of the invention have been described with reference to different subject matters. In particular, some embodiments have been described with reference to apparatus type claims whereas other embodiments have been described with reference to method type claims. However, a person skilled in the art will gather from the above and the following description that, unless other notified, in addition to any combination of features belonging to one type of subject matter also any combination between features relating to different subject matters, in particular between features of the apparatus type claims and features of the method type claims is considered as to be disclosed with this application.

Brief Description of the Drawings

The aspects defined above and further aspects of the present invention are apparent from the examples of embodiment to be described hereinafter and are explained with reference to the examples of embodiment. The invention will be described in more detail hereinafter with reference to examples of embodiment but to which the invention is not limited.

For a better understanding of the invention figures are given. These figures show:

Fig. 1: a regular beam arrangement of 20 beams through the cross section: the two layers have a 45° angle relative to each other

Fig. 2: a regular beam arrangement of 20 beams through the cross section: the two layers have a 30° angle relative to each other

Fig. 3: a regular beam arrangement of 20 beams through the cross section: the two layers have a 60° angle relative to each other

Fig. 4: CAD sectional model showing the structure of the inner pipe 1a and 1b with holes 2a and 2b as well as the outer ring 3a and 3b

Fig. 5: CAD model showing the structure of the inner pipe 1a and the outer ring 3a with the sender and detector mounting

Fig. 6: CAD model showing the structure of the inner pipe 1a with holes 2a and the outer ring 3a

Fig. 7: Overall CAD model view showing all relevant components of the invention

Detailed Description of Exemplary Embodiments

The illustrations in the drawings are schematic. It is noted that in different figures similar or identical elements are provided with the same reference signs.

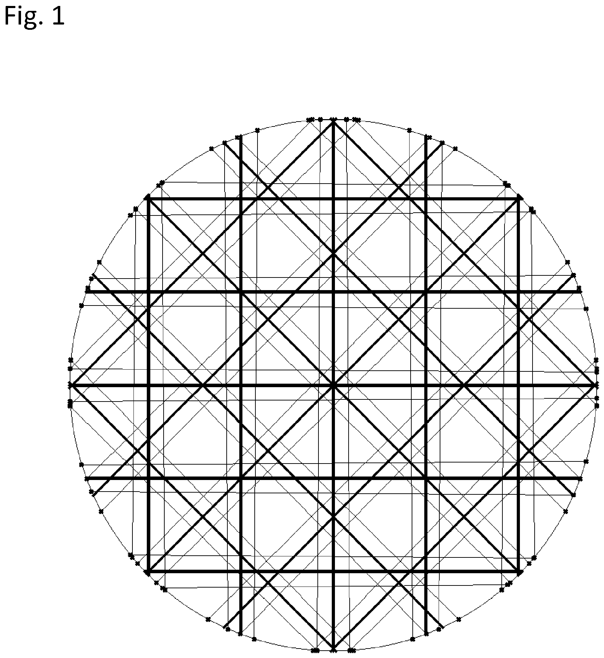

**Fig. 1 to Fig. 3** shows a projection of a first optical measurement layer within a first inner pipe and a second optical measurement layer within the second inner pipe. Within the first optical measurement layer, first light beams run between a first inlet hole of the first inner pipe and a first outlet hole of the first inner pipe. Accordingly, second light beams run between a second inlet hole of the second inner pipe and the second outlet hole of the second inner pipe.

In Fig. 1, a regular beam arrangement of 20 beams through the cross section, i.e. the respective first and second optical measurement layers. The two optical measurement layers and the respective first light beams and second light beams have a 45° angle relative to each other.

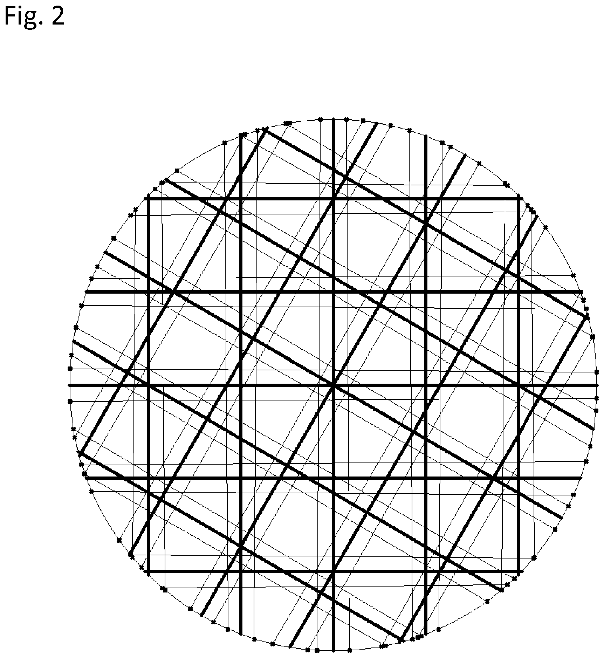

In Fig. 2, the two optical measurement layers and the respective first light beams and second light beams have a 30° angle relative to each other.

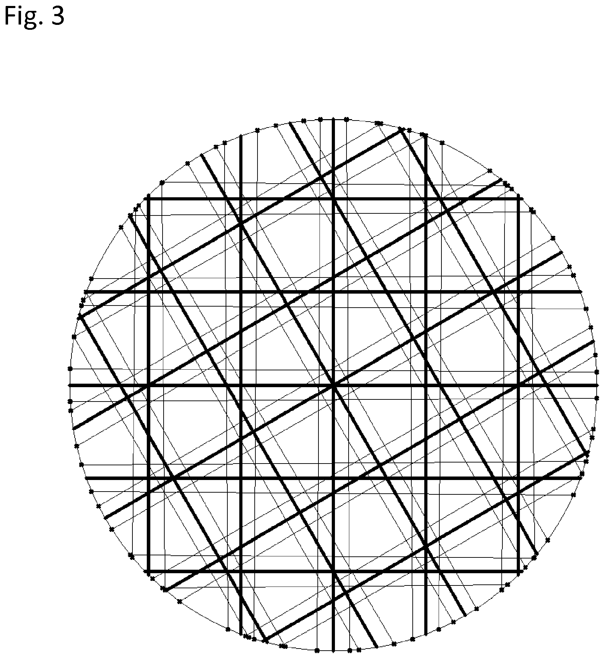

In Fig. 3, the two optical measurement layers and the respective first light beams and second light beams have a 60° angle relative to each other.

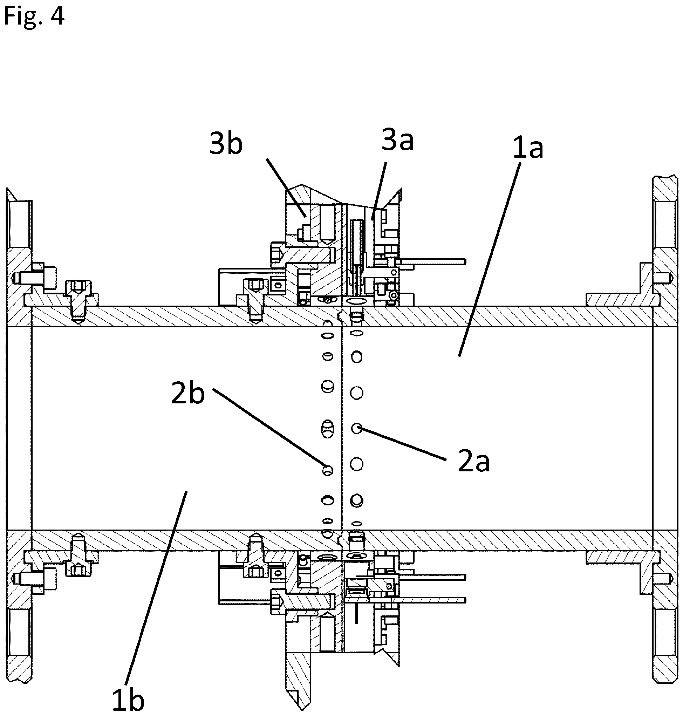

**Fig. 4** shows a schematic view according to an exemplary embodiment of the present invention. Fig. 4 shoes the structure of the inner pipes 1a and 1b with respective holes 2a and 2b as well as the outer rings 3a and 3b.

The device shown comprises two optical measurement layers providing different positions for optical paths that can be rotated relative to each other with discrete angles between the two optical measurement layers and the light beams, respectively (see Figs 1 to 3).

Each measurement layer consists of and can be provided by a respective pipe 1a, 1b. A first inner pipe 1a contains a first optical measurement layer and a second inner pipe 1b contains a second optical measurement layer.

For forming the respective optical measurement layer, each of the respective first inner pipe 1a and second inner pipe 1b comprise respective holes 2a, 2b for providing an optical access through the respective first and second inner pipes 1a, 1b. Furthermore, respective lenses may be provided in the path of the light beams. Hence, through the holes 2a, 2b, multiple light beams extend through the respective first and second inner pipes 1a, 1b from light sources through the respective first and second inner pipes 1a, 1b to respective detectors.

For example, the first inner pipe 1a comprises a line of holes 2a, such that the holes 2a are formed spaced apart along a circumferential direction of the first inner pipe 1a. The circumferential direction is perpendicular to an axial direction of the respective pipe 1a, 1b and to a radial direction of the respective pipe 1a, 1b. Specifically, in the first inner pipe 1a a first hole 2a may form an inlet hole through which a respective light beam may enter the first inner pipe 1a and a second hole 2a, which is formed in the first inner pipe 1a opposed to the first hole 2a, may form an outlet hole through which the respective light beam, which entered the first inlet hole, exits the first inner pipe 1a. Hence, a respective light source may be arranged at or close to the first inlet hole 2a, wherein a respective light sensor is arranged at or close to the first outlet hole 2a for receiving the light beam which has been emitted by the light source through the first inlet hole 2a.

Accordingly, the second inner pipe 1b comprises a line of holes 2b, such that the holes 2b are formed spaced apart along a circumferential direction of the second inner pipe 1b. Specifically, in the second inner pipe 1b a second hole 2b may form an inlet hole through which a respective light beam may enter the second inner pipe 1b and a further second hole 2b, which is formed in the second inner pipe 1b opposed to the second hole 2b, may form an outlet hole through which the respective light beam, which entered the second inlet hole 2b, exits the second inner pipe 1b. Hence, a respective light source may be arranged at or close to the second inlet hole 2b, wherein a respective light sensor/detector is arranged at or close to the second outlet hole 2b for receiving the light beam which has been emitted by the light source through the second inlet hole 2b.

Furthermore, a first outer ring 3a and a second outer ring 3b is provided. The first outer ring 3a is mounted to the first inner pipe 1a and the second outer ring 3b is mounted to the second inner pipe 1b. The respective outer rings 3a, 3b are equipped with light sources and sensors arranged around (i.e. along the circumferential direction) the first and second inner pipe 1a, 1b allowing light to pass the cross section (i.e. the layer) of the respective inner pipes 1a, 1b, in particular between an inlet hole 2a, 2b and an opposing outlet hole 2a, 2b.

The first outer ring 3a together with the first inner pipe 1a may be rotated and turned around a circumferential direction with respect to the second outer ring 3b together with the second inner pipe 1b. Hence, the angular arrangement of the respective first holes 2a with respect to the respective second holes 2b may be adjusted accordingly.

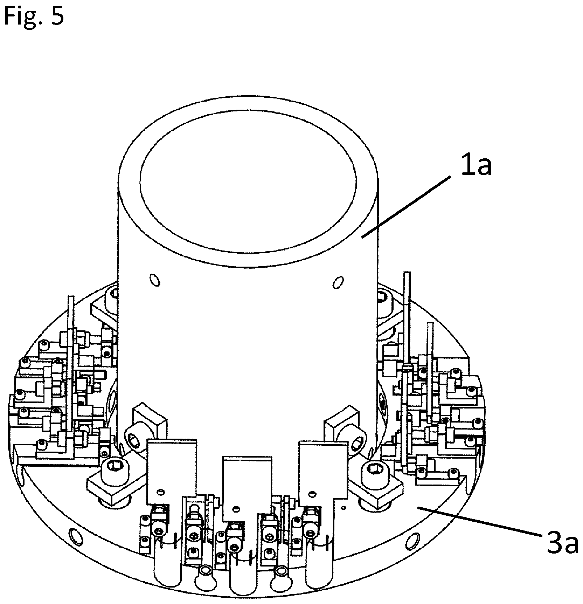

**Fig. 5** shows a schematic view of a CAD model according to an exemplary embodiment of the present invention showing the structure of the inner pipe 1a and the outer ring 3a with a sender and a detector mounted to the outer ring 3a. The sender and the detector are arranged in such a way, that a light beam can be emitted by the sender through an inlet hole 2a and the light beam exits through an outlet hole 2a, wherein the detector is arranged at the outlet hole 2a for detecting the light beam.

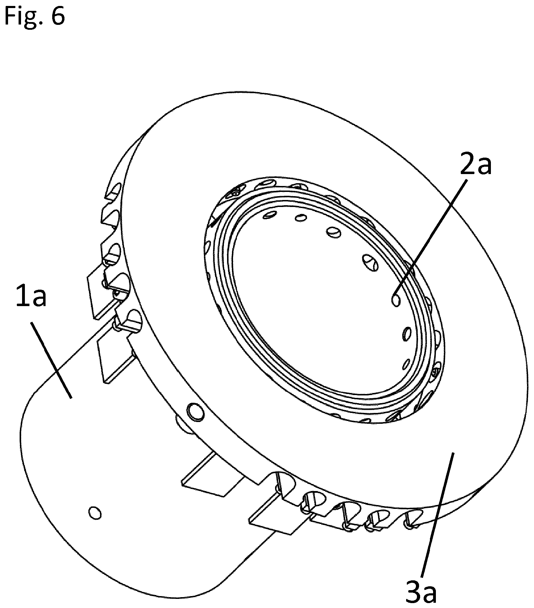

**Fig. 6** shows a further schematic view of a CAD model showing the structure of the inner pipe 1a with holes 2a and the outer ring 3a

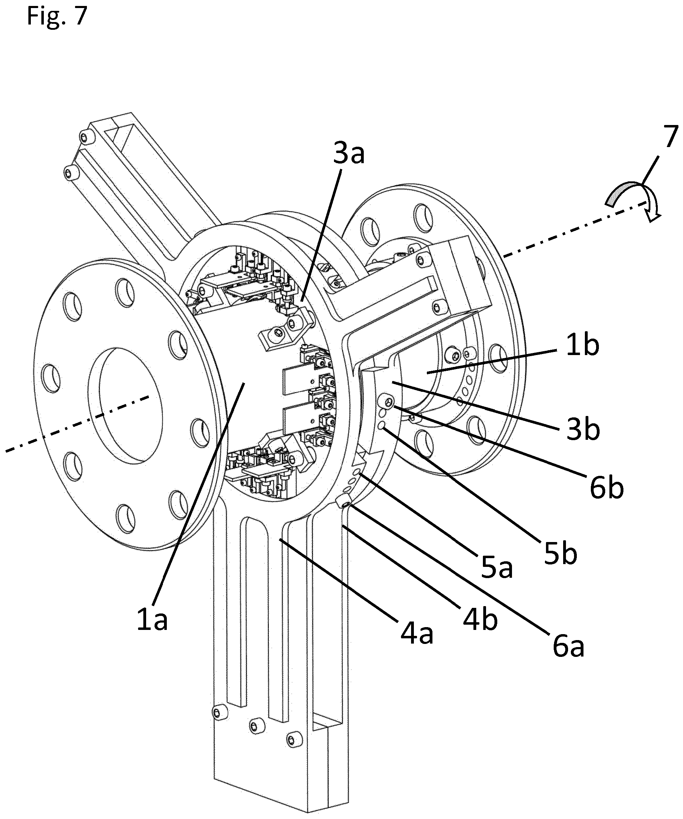

**Fig. 7** shows a schematic view of a CAD model view showing components of the device according to an exemplary embodiment of the present invention.

A first mounting ring 4a and a second mounting ring 4b are provided. The two mounting rings 4a, 4b connect and fix the respective two inner pipes 1a, 1b and allows to rotate one or both inner pipes 1a, 1b together with the outer rings 3a, 3b with discrete angles relative to each other according to the discrete positions of respective mounting holes 5a, 5b in the mounting rings 4a, 4b in order to optimize projections and improve tomographic reconstruction.

In other words, the first outer ring 3a to which the first inner pipe 1a is mounted, can be fixed to the first mounting ring 4a. The second outer ring 3b, to which the second inner pipe 1b is mounted, can be fixed to the second mounting ring 4b. The first outer ring 3a can be fixed to the first mounting ring 4a with different predefined circumferential orientations. In other words, the first outer ring 3a can be rotated and in a predefined rotational orientation, the first outer ring 3a together with the first inner pipe 1a can be fixed to the first mounting ring 4a. Accordingly, the second outer ring 3b can be fixed to the second mounting ring 4b with different predefined circumferential orientation. Hence, the first outer ring 3a and the second outer ring 3b can be adjusted and orientated with respect to each other along its circumferential direction such that also both inner pipes 1a, 1b may be rotated with respect to each other until a desired circumferential orientation of the respective pipes 1a, 1b is fulfilled. Hence, the angles between a first light beam running through a first pair of holes 2a in the first inner pipe 1a and a second light beam running through a second pair of holes 1b in the second inner pipe 1b can be adjusted in order to optimize projections and improve tomographic reconstruction.

The first mounting ring 4a and the second mounting ring 4b may be monolithically formed. Alternatively, the first mounting ring 4a and the second mounting ring 4b may be detachably fixed together, for example by screw connections as shown in Fig. 7.

Furthermore, the first mounting ring 4a comprises first adjustment holes 5a. The outer ring 3a comprises one receiving hole so that an adjustment screw 6a may fix the receiving hole to one of the adjustment holes 5a. The adjustment holes 5a are arranged spaced apart from each other along the circumferential direction 7. Hence, the angular adjustment of the outer ring 3a can be adjusted by fixing the receiving hole by the adjustment screw 6a to a respective adjustment hole 5a. Accordingly, the second mounting ring 4b comprises second adjustment holes 5b. The outer ring 3b comprises one receiving hole so that a further adjustment screw 6b may fix the receiving hole of the second outer ring 3b to one of the adjustment holes 5b. The adjustment holes 5b are arranged spaced apart from each other along the circumferential direction 7.

It should be noted that the term "comprising" does not exclude other elements or steps and "a" or "an" does not exclude a plurality. Also elements described in association with different embodiments may be combined. It should also be noted that reference signs in the claims should not be construed as limiting the scope of the claims.

## Claims

1. Device to determine the gas concentration distribution in a flow based on non-dispersive absorption spectroscopy comprising two optical measurement layers providing different positions for optical paths that can be rotated relative to each other with discrete angles between the two layers, the device further comprises a pipe comprising a first inner pipe (1a) for forming the one of the optical measurement layers and a second inner pipe (1b) for forming the other one of the optical measurement layers, each inner pipe (1a, 1b) comprising respective holes (2a, 2b) and lenses for providing an optical access through the respective inner pipes (1a, 1b) and the respective layers, the lenses allowing light to pass through the holes (2a, 2b) from outside the respective inner pipes (1a, 1b), pass the cross section of the respective inner pipes (1a, 1b) and pass opposite holes (2a, 2b),
a first outer ring (3a) and a second outer ring (3b),
wherein the first inner pipe (1a) is mounted to the first outer ring (3a) and the second inner pipe (1b) is mounted to the second outer ring (3b),
wherein each outer ring (3a, 3b) comprises light sources and sensors arranged around the inner pipes (1a, 1b) allowing light to pass a respective cross section of the respective inner pipes (1a, 1b),
a first and a second mounting ring (4a, 4b),
wherein the first outer ring (3a) is rotatable in a predefined rotational orientation and fixable together with the first inner pipe (1a) to the first mounting ring (4a), wherein the second outer ring (3b) is fixable to the second mounting ring (4b), such that the mounting rings (4a, 4b) connect the two inner pipes (1a, 1b) and allow to rotate one or both inner pipes (1a, 1b) with discrete angles relative to each other according to the discrete positions of adjustment holes (5a, 5b) in the mounting rings (4a, 4b).

2. Device according to claim 1,
wherein the holes (2a, 2b) in a respective line of holes (2a, 2b) are arranged spaced apart from each other along a circumferential direction of respective inner pipe (1a, 1b).

3. Device according to claim 1 or 2,
wherein the respective holes (2a, 2b) have different hole diameters with respect to each other.

4. Device according to one of the claims 1 to 3,
wherein the first mounting ring (4a) comprises first adjustment holes (5a) being arranged spaced apart from each other along a circumferential direction of the respective inner pipe (1a),
wherein the outer ring (3a) comprises one receiving hole for receiving an adjustment screw (6a) for fixing the receiving hole to one of the adjustment holes (5a), so that an angular fixation of the outer ring (3a) is adjustable by fixing the receiving hole by the adjustment screw (6a) one of the respective adjustment holes (5a).

**Patentansprüche**

1. Eine Vorrichtung zum Bestimmen der Gaskonzentrationsverteilung in einer Strömung basierend auf nicht-dispersiver Absorptionsspektroskopie, aufweisend zwei optische Messschichten, welche verschiedene Positionen für optische Pfade bereitstellen, welche relativ zueinander gedreht werden können, mit diskreten Winkeln zwischen den zwei Schichten, wobei die Vorrichtung ferner aufweist
eine Leitung aufweisend eine erste innere Leitung (1a) zum Bilden der einen der optischen Messschichten und eine zweite innere Leitung (1b) zum Bilden der anderen der optischen Messschichten, wobei jede innere Leitung (1a, 1b) jeweilige Öffnungen (2a, 2b) und Linsen zum Bereitstellen eines optischen Zugangs durch die jeweiligen inneren Leitungen (1a, 1b) und der jeweiligen Schichten aufweist, wobei die Linsen ermöglichen, dass Licht von außerhalb der jeweiligen inneren Leitungen (1a, 1b) die Öffnungen (2a, 2b), den Querschnitt der jeweiligen inneren Leitungen (1a, 1b), und gegenüberliegende Öffnungen (2a, 2b) passiert,
einen ersten äußeren Ring (3a) und einen zweiten äußeren Ring (3b), wobei die erste innere Leitung (1a) an den ersten äußeren Ring (3a) montiert ist und die zweite innere Leitung (1b) an den zweiten äußeren Ring (3b) montiert ist,
wobei jeder äußere Ring (3a, 3b) Lichtquellen und Sensoren aufweist, welche um die inneren Leitungen (1a, 1b) angeordnet sind, welche es ermöglichen, dass Licht einen jeweiligen Querschnitt der jeweiligen inneren Leitungen (1a, 1b) passiert,
einen ersten und einen zweiten Montagering (4a, 4b),
wobei der erste äußere Ring (3a) in einer vordefinierten Drehorientierung drehbar ist und zusammen mit der ersten inneren Leitung (1a) an dem ersten Montagering (4a) fixierbar ist,
wobei der zweite äußere Ring (3b) an dem zweiten Montagering (4b) fixierbar ist, so dass die Montageringe (4a, 4b) die zwei inneren Leitungen (1a, 1b) verbinden und ermöglichen, eine oder beide innere Leitungen (1a, 1b) mit diskreten Winkeln relativ zueinander gemäß den diskreten Positionen von Justieröffnungen (5a, 5b) in den Montageringen (4a, 4b) zu drehen.

2. Die Vorrichtung gemäß Anspruch 1,

wobei die Öffnungen (2a, 2b) in einer jeweiligen Linie von Öffnungen (2a, 2b) entlang einer Umfangsrichtung der jeweiligen inneren Leitung (1a, 1b) voneinander beabstandet angeordnet sind.

3. Die Vorrichtung gemäß Anspruch 1 oder 2,
wobei die jeweiligen Öffnungen (2a, 2b) verschiedene Öffnungsdurchmesser in Bezug aufeinander haben.

4. Die Vorrichtung gemäß einem der Ansprüche 1 bis 3,
wobei der erste Montagering (4a) erste Justieröffnungen (5a) aufweist, welche beabstandet voneinander entlang einer Umfangsrichtung der jeweiligen inneren Leitung (1a) angeordnet sind,
wobei der äußere Ring (3a) eine Aufnahmeöffnung aufweist, zum Aufnehmen einer Justierschraube (6a) zum Fixieren der Aufnahmeöffnung an einer der Justieröffnungen (5a), so dass eine Winkelfixierung des äußeren Rings (3a) einstellbar ist, mittels Fixierens der Aufnahmeöffnung mittels der Justierschraube (6a) an einer der jeweiligen Justieröffnungen (5a).

**Revendications**

1. Dispositif pour déterminer la distribution de concentration de gaz dans un flux sur la base d'une spectroscopie d'absorption non dispersive comprenant deux couches de mesure optique fournissant des positions différentes pour des chemins optiques qui peuvent être tournés l'un par rapport à l'autre selon des angles distincts entre les deux couches, le dispositif comprend en outre
un tuyau comprenant un premier tuyau intérieur (1a) pour former l'une des couches de mesure optique et un deuxième tuyau intérieur (1b) pour former l'autre des couches de mesure optique, chaque tuyau intérieur (1a, 1b) comprenant des trous respectifs (2a, 2b) et des lentilles pour fournir un accès optique à travers les tuyaux intérieurs respectifs (1a, 1b) et les couches respectives, les lentilles permettant à la lumière de passer à travers les trous (2a, 2b) depuis l'extérieur des tuyaux intérieurs respectifs (1a, 1b), de passer la section transversale des tuyaux intérieurs respectifs (1a, 1b) et de passer des trous opposés (2a, 2b),
un premier anneau extérieur (3a) et un deuxième anneau extérieur (3b), le premier tuyau intérieur (1a) étant monté sur le premier anneau extérieur (3a) et le deuxième tuyau intérieur (1b) étant monté sur le deuxième anneau extérieur (3b),
chaque anneau extérieur (3a, 3b) comprenant des sources de lumière et des capteurs disposés autour des tuyaux intérieurs (1a, 1b) permettant à la lumière de passer une section transversale respective des tuyaux intérieurs respectifs (1a, 1b),
un premier et un deuxième anneaux de montage (4a, 4b),
le premier anneau extérieur (3a) étant apte à tourner dans une orientation de rotation prédéfinie et étant apte à être fixé avec le premier tuyau intérieur (1a) au premier anneau de montage (4a), le deuxième anneau extérieur (3b) étant apte à être fixé au deuxième anneau de montage (4b), de sorte que les anneaux de montage (4a, 4b) relient les deux tuyaux intérieurs (1a, 1b) et permettent de faire tourner un ou les deux tuyaux intérieurs (1a, 1b) selon des angles distincts l'un par rapport à l'autre selon les positions distinctes de trous de réglage (5a, 5b) dans les anneaux de montage (4a, 4b).

2. Dispositif selon la revendication 1,
dans lequel les trous (2a, 2b) dans une ligne respective de trous (2a, 2b) sont agencés de façon espacée les uns des autres le long d'une direction circonférentielle du tuyau intérieur respectif (1a, 1b).

3. Dispositif selon la revendication 1 ou la revendication 2,
dans lequel les trous respectifs (2a, 2b) ont des diamètres de trous différents les uns par rapport aux autres.

4. Dispositif selon l'une des revendications 1 à 3,
dans lequel le premier anneau de montage (4a) comprend des premiers trous de réglage (5a) agencés de façon espacée les uns des autres le long d'une direction circonférentielle du tuyau intérieur respectif (1a),
l'anneau extérieur (3a) comprenant un trou de réception pour recevoir une vis de réglage (6a) pour fixer le trou de réception à l'un des trous de réglage (5a), de sorte qu'une fixation angulaire de l'anneau extérieur (3a) soit réglable en fixant le trou de réception par la vis de réglage (6a) à l'un des trous de réglage (5a) respectifs.

Fig. 1

Fig. 2

Fig. 3

Fig. 4

Fig. 5

1a

3a

Fig. 6

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6593573 B1
- US 20100241361 A1
- US 20150355080 A1
- CN 104280355
- CN 106442403
- KR 101839704 B1
- WO 2009052157 A1
- US 5798840 A

### Non-patent literature cited in the description

- Measuring ammonia - Development and application of measurement techniques for the detection of ammonia. **HOLE, O.M.** Master thesis. University of Lund, 2012
- **DAUN, K. J.** Infrared species limited data tomography through Tikhonov reconstruction. *Journal of Quantitative Spectroscopy and Radiative Transfer,* 2010, vol. 111, 105-115
- **J. SONG et al.** Tomography system for measurement of gas properties in combustion flow field. *Chinese J. Aeronaut.,* 2017, vol. 30 (5), 1697-1707